# EUROPÄISCHE PATENTANMELDUNG

(11) **EP 4 191 601 A1**
(43) Veröffentlichungstag der Anmeldung: **07.06.2023**
(21) Anmeldenummer: 23153223.5
(22) Anmeldetag: 13.01.2015
(51) Int. Cl.: G16H 20/30, G16H 40/63, G16H 40/67

(54) **SYSTEM ZUR UNTERSTÜTZUNG EINES LAIENHELFERS BEI DER REANIMATION EINES PATIENTEN MIT KREISLAUFSTILLSTAND**

(30) Priorität: 22.01.2014 DE 102014100710; 27.02.2014 DE 102014102590
(62) Teilanmeldung aus: 15709084.6
(71) Anmelder: Müller, Michael, 77815 Bühl (DE); Roth, Matthias, 79111 Freiburg (DE); Schorling, Per, 5700 Svendborg (DK)
(72) Erfinder: Müller, Michael, 77815 Bühl (DE)
(74) Vertreter: Werner, André

(57) **Zusammenfassung**

Die Erfindung betrifft ein System zur Unterstützung eines Helfers bei der Durchführung von Thoraxkompressionen zur zeitnahen Reanimation einer von einem Kreislaufstillstand betroffenen Person (1), wobei der Helfer insbesondere ein unausgebildeter Laienhelfer (8) sein kann. Zur Erkennung des Kreislaufzustandes der Person (1) und Vermittlung unterstützender Erstmaßnahmen ist ein mit einem ähnlich einem Heftpflaster (5) ausgebildeten Trägermittel verbundener Lagesensor (4) durch Kleben an entsprechender Position am Brustbein (2) der Person (1) angeordnet. Der als Beschleunigungssensor ausgebildete Lagesensor (4) ist außerdem drahtlos oder kabelgebunden mit einem mobilen Endgerät (6) verbunden. Dieses Endgerät (6) ist als Smartphone ausgebildet, wobei das Smartphone mit einem entsprechenden Anwendungsprogramm ausgerüstet ist. Die Erfindung hat den Vorteil, dass bei ihrer Verwendung an einem von einem Kreislaufstillstand betroffenen Patienten (1) auch ein Laienhelfer (8) in der Lage ist, den betroffenen Patienten (1) zeitnah zu reanimieren, um schwerwiegende Schädigungen zu vermeiden beziehungsweise sein Leben zu erhalten.

## Beschreibung

Die Erfindung betrifft ein System zur Unterstützung eines Laienhelfers bei der Durchführung erster Hilfsmaßnahmen zur Reanimation einer Person beziehungsweise eines Patienten mit Kreislaufstillstand.

Der plötzliche Herztod ist eine der häufigsten Todesursachen. Trotz erheblicher Bemühungen in den letzten Dekaden sowie der Etablierung internationaler Leitlinien für die Behandlung des Herzkreislaufstillstands ist die Überlebensrate unter 10 %. Die meisten der 75.000 Menschen, die jährlich in Deutschland einen Kreislaufstillstand erleiden, sterben oder überleben mit bleibenden neurologischen Schäden und sind dauerhaft pflegebedürftig. Die wesentlichen Probleme beim Kampf gegen den plötzlichen Herztod sind die folgenden:
Überleben nach plötzlichem Herztod ist nur möglich, wenn innerhalb der ersten Minuten und noch vor dem Eintreffen des Rettungsdienstes mit der Herzdruckmassage begonnen wird und diese mit ausreichender Qualität erfolgt.
Dabei ist das Ziel der Massage, dass diese entsprechend der jeweils aktuellen Empfehlungen der Leitlinien erfolgt. Momentan empfehlen diese eine Eindrucktiefe von 5 - 6 cm und eine Frequenz von beispielsweise 100 / min sowie eine komplette Entlastung nach jeder Thorakompression. Die Unterbrechungen der Herzdruckmassage - um beispielsweise zu beatmen oder einen Defibrillationsschock auszulösen - sollen so kurz wie möglich sein, damit das Gehirn möglichst ununterbrochen durchblutet wird und keine irreversiblen Schäden auftreten.

In Deutschland leiten nur in etwa 20 % der Fälle Laienhelfer die wichtigen Maßnahmen der Wiederbelebung (vor allem Herzdruckmassage) ein, in anderen europäischen Ländern liegt diese Rate bei bis zu 70 %. Die Qualität der Herzdruckmassage nach Schulungsmaßnahmen zu den Grundlagen ist gut, nimmt aber bereits nach sechs Monaten wieder erheblich ab. Während der Wiederbelebung sinkt zudem die Qualität der Herzdruckmassage bereits nach zwei Minuten erheblich ab.

Die Überlebenswahrscheinlichkeit eines Patienten mit Kreislaufstillstand hängt wesentlich von den Maßnahmen des Laienhelfers ab. Dieser ist jedoch mit der Situation häufig überfordert und nicht ausreichend trainiert.

Nach dem internen Stand der Technik ist ein Verfahren zur Defibrillation während der Durchführung einer Thoraxkompression durch einen Helfer sowie ein dazu eingesetzter Defibrillator bekannt, wobei die Anzahl der durchgeführten Thoraxkompressionen ermittelt und jeweils nach einer definierten Anzahl von Kompressionen nach Beginn der Durchführung beziehungsweise nach dem zuletzt erfolgten Defibrillatorschock, ein weiterer Defibrillatorschock abgegeben wird, wobei die Thoraxkompressionen während der Abgabe der Defibrillatorschocks weitergeführt werden können.

Weiterhin ist aus dem internen Stand der Technik ein Defibrillatorsystem mit einer Kontrolleinheit bekannt, über die der Defibrillator gesteuert werden kann und mit der für die Reanimation relevante Daten angezeigt werden können. Als Kontrolleinheit ist eine bidirektionale Brille eingesetzt, die eine Steuerung der Kontrolleinheit durch Bewegung der Augen (Eye-Tracking) ermöglicht.

Außerdem ist ein Ultraschallsystem und ein Verfahren zur Kommunikation zwischen einem Ultraschallgerät und einer bidirektionalen Datenbrille bekannt, wobei die Datenbrille ein Ultraschallbild auf einem transparenten Display wiedergibt, während die Datenbrille dem Anwender die gleichzeitige Betrachtung die Stelle des Eingriffs am Patienten ermöglicht. Das Display beinhaltet zusätzlich eine Einrichtung zu einer Verfolgung der Augenbewegung (Eye-Tracking), wodurch die Datenbrille als Steuereinheit für das Ultraschallgerät ausgebildet ist. Durch die Verbindung der Datenbrille mit einem Überwachungsmonitor werden die als Vitalparameter bezeichneten wichtigen Werte über den Zustand des Patienten in die Datenbrille übertragen.

Aus EP 1 128 795 B1 ist ein System zum Messen und Veranlassen von Brustkompressionen, bekannt. Es umfasst einen mobilen CPR-Kompressionsmonitor (CPR - Cardiopulmonale Reanimation, Herz-Lungen-Wiederbelebung) zur Überwachung der Thoraxkompressionen zur Reanimation einer von einem Kreislaufstillstand betroffenen Person. Das Gerät wird auf der Hand des Helfers oder auf dem Patienten abgelegt und umfasst Beschleunigungssensoren sowie eine Schnittstelle zur Datenübertragung. An diese Schnittstelle wird über ein Kabel eine Auswerteeinheit mit Bildschirm angeschlossen. Diese Auswerteeinheit mit Bildschirm kann im CPR-Kompressions-monitor integriert oder ein eigenständiges Gerät sein. Dieses System ist für geschultes und erfahrenes medizinisches Personal konzipiert.

In WO 2011/156374 A3 ist ein drahtloses Ultraschall-GesundheitszustandsÜberwachungssystem offenbart. Das System, umfassend eine Sensoreinheit mit Konverterlogik und Schalltransmitter, zeichnet physiologische Signale auf und überträgt diese mittels Schall/Ultraschall zu einem Empfangsgerät (Smartphone oder Computer). Die Sensoreinheit, aufweisend mehrere Elektroden, ist an einer Schutzhülle für ein Smartphone angeordnet. Mithilfe der die Haut des Patienten kontaktierenden Elektroden wird ein EKG (EKG - ElektroKardioGramm, Aufzeichnung des Herzrhythmus) aufgezeichnet und mittels Schall an einen Empfänger, z. B. ein in die Schutzhülle gestecktes Smartphone, übermittelt.

US 8,509,882 B2 beschreibt eine Software-Applikation (App) für ein Smartphone, die per Schall übermittelte EKG-Signale darstellen und auswerten kann. Zusätzlich können Sprache, GPS-Daten oder Bewegungen des Smartphones aufgezeichnet werden. Die gesammelten Daten können auf einen Webserver übertragen werden.

WO 2006/104977 A2, EP 2 255 845 A1 und DE 60 2004 002 147 T2 offenbaren ein professionelles medizinisches System zur Unterstützung eines Ersthelfers, wobei dieser in der Reanimation geschult sein muss. Das System umfasst unter anderem einen Defibrillator und ein mobiles Anzeige- und Steuergerät.

Desgleichen beschreibt EP 1 858 472 B1 ein zwar mobiles, doch komplexes medizinisches System zur Unterstützung eines Helfers bei der Reanimation, das auch einen Defibrillator umfasst. Es ist vorgesehen, das Gerät an wenigen zentralen Orten mit hohem Menschenaufkommen zu stationieren, damit Ersthelfer einen schnellen Zugriff darauf erhalten. Allerdings ist auch dieses Gerät nur von geschulten Helfern einigermaßen sinnvoll einsetzbar.

Verfahren und Vorrichtungen zur exakten Bestimmung der Eindrucktiefe bei Brustkorbkompressionen sind in WO 2004/037 154 A2 und DE 11 2010 000 978 T5 gezeigt.

Wesentliche Nachteile bei den nach dem Stand der Technik bekannten Lösungen bestehen darin, dass eine Anwendung entsprechender Hilfsmaßnahmen zeitaufwendig und der jeweilige (Erst-)Helfer häufig überfordert sowie nicht ausreichend trainiert ist. Zudem fehlt ein hohes Maß an Kenntnissen, um eine erfolgreiche Notbehandlung des jeweiligen Patienten durchführen zu können

Speziell bei Systemen mit automatischem Defibrillator hat sich zudem gezeigt, dass bis zum Beginn der ersten Herzdruckmassage vergleichsweise viel Zeit - im Mittel knapp zwei Minuten - aufgrund vorbereitender Maßnahmen verloren geht (siehe z. B.: M.P. Müller et al, "An AED Is Not An AED", präsentiert auf der American Heart Assoc. ReSS 2014, 7.-10. Nov. 2014, Chicago, USA). Insbesondere zu Beginn der Notfallbehandlung müssen erst Elektroden fixiert, der automatische Defibrillator gestartet und (automatisierte) Messungen und Analysen durchgeführt werden, wobei während dieser Zeit keine lebensrettende Herzdruckmassage erfolgt. Dies steht jedoch im krassen Widerspruch zu den Empfehlungen für wiederbelebende Maßnahmen, wonach schnellstmöglich, d. h. nach weniger als 30 Sekunden, eine Herzdruckmassage durchzuführen ist.

Der Erfindung liegt die Aufgabe zugrunde, eine Lösung zu schaffen die es ermöglicht, einen ungeschulten Laienhelfer zu sofortigen ersten lebensrettenden Maßnahmen einer von einem Kreislaufstillstand betroffenen Person, nachfolgend Patient genannt, anzuleiten und durch wesentliche Informationen über Art der durchzuführenden und Erfolg der durchgeführten Maßnahmen zur Reanimation fachgerecht zu unterstützen, wobei das Ziel ist, irreversible Schädigungen des Patienten durch Herzstillstand bis zum Eintreffen medizinisch geschulter Helfer zu vermeiden.

Die Aufgabe wird gemäß der Erfindung mit den Merkmalen nach dem Patentanspruch 1 gelöst; vorteilhafte Weiterbildungen der Erfindung sind in den Unteransprüchen 2 bis 7 beschrieben.

Gemäß der Erfindung wird ein System zur Anleitung und Unterstützung (auch als "Unterstützungssystem" bezeichnet) eines Helfers, der insbesondere ein in der Reanimation unausgebildeter Laienhelfer ist, bei der Reanimation des Patienten mit einem Kreislaufstillstand bereitgestellt. Das Unterstützungssystem umfasst ein mobiles Endgerät, eine Software-Applikation (d. h. ein computerausführbares Programm) für dieses Endgerät und einen Lagesensor mit einem Trägermittel.

Das mobile Endgerät ist derart ausgestaltet, dass auf dem Endgerät eigenständige Software-Applikationen ausführbar sind. Vorzugsweise ist dieses mobile Endgerät ein typischerweise von Passanten bei der täglichen Routine mitgeführtes, elektronisches Gerät. Das mobile Endgerät kann jede Art von tragbarem Klein- oder Kleinstcomputer, zum Beispiel ein Smartphone, ein Phablet, ein Tablet oder ein Netbook, sein.

Das mobile Endgerät ist mittels einer auf dem Gerät ausführbaren Software-Applikation, zum Beispiel in Form einer sogenannten App, derart ausgebildet, dass es den Helfer bei den zur Reanimation nötigen Maßnahmen - insbesondere dem Erkennen des Kreislaufzustandes des Patienten, Absetzen eines Notrufs und Durchführen physischer Hilfsmaßnahmen - unterstützt, sodass es insbesondere zur Unterstützung von unerfahrenen Laienhelfern geeignet ist.

Das mobile Endgerät ist vorzugsweise ein Smartphone, d. h. ein Kleinstcomputer mit Telefoniefunktion, entsprechend ist die Software-Applikation eine Smartphone-App.

Das mobile Endgerät kann mittels der Software-Applikation derart ausgebildet sein, dass in einem ersten Schritt eine Abfrage von Symptomen (des zu behandelnden Patienten) durchgeführt wird, wobei von dem Endgerät anhand der Ergebnisse der Abfrage ermittelt wird, ob bei dem Patienten tatsächlich ein Kreislaufstillstand vorliegt oder nicht. So können in diesem ersten Schritt z. B. in der Software-Applikation sehr einfache und auch für Laien verständliche Abfragen nach den Symptomen erfolgen, um festzustellen, ob ein Kreislaufstillstand vorliegt.

Des Weiteren können das mobile Endgerät und die Software-Applikation derart ausgebildet sein, dass automatisiert ein Notruf abgesetzt wird, z. B. an die zuständige Rettungsleitstelle. Der Notruf kann z. B. in Form einer Audio-Nachricht oder einer Text-Nachricht abgesetzt werden. Das mobile Endgerät und die Software-Applikation können derart ausgebildet sein, dass das Absetzen des Notrufs in Abhängigkeit von dem Ergebnis der Symptom-Abfrage erfolgt (wobei ein Notruf lediglich dann abgesetzt wird, wenn die Symptom-Abfrage ergeben hat, dass tatsächlich ein Kreislaufstillstand vorliegt). Es kann jedoch auch vorgesehen sein, dass das Absetzen des Notrufs unabhängig von dem Ergebnis der Symptom-Abfrage erfolgt, sodass das Absetzen des Notrufs nicht durch das Durchlaufen der SymptomAbfrage zeitlich verzögert wird. Im letzteren Fall kann z. B. vorgesehen sein, den Notruf zeitgleich mit der Aktivierung der Symptom-Abfrage automatisiert abzusetzen, wobei das Absetzen des Notrufs eben durch die Aktivierung der Symptom-Abfrage ausgelöst wird.

In vorteilhafter Weise kann die Software-Applikation über eine Schnittstelle im Betriebssystem des mobilen Endgeräts auf Positionierungsdaten bzw. Ortskoordinaten zugreifen. Das mobile Endgerät kann beispielsweise einen Empfänger (z. B. GPS-Empfänger) für ein (z. B. satellitengestütztes) Navigations- bzw. Positionsbestimmungssystem (z. B. das Global Positioning System GPS) aufweisen und derart ausgebildet sein, dass von ihm mittels des Empfängers die aktuelle Position des Endgeräts ermittelt wird. Das mobile Endgerät kann auch derart ausgebildet sein, dass über eine Triangulation von mit dem mobilen Endgerät in Verbindung stehenden Funkmasten (z. B. Mobilfunk-Antennen) eine Ortsbestimmung möglich ist. Es kann vorgesehen sein, dass diese Positionskoordinaten weitergeleitet werden, z. B. als Bestandteil des Notrufs. Somit kann die Position des Helfers mittels des Notrufs ohne zusätzlichen Aufwand bzw. Eingreifens seitens des Helfers an die zuständige Rettungsleitstelle übermittelt werden.

Das Endgerät kann zudem derart ausgebildet bzw. programmiert sein, dass von ihm z. B. visuell über eine optische Anzeige des Endgerätes und/oder akustisch über eine Lautsprechereinrichtung des Endgerätes eine Anleitung des Helfers zur Reanimation des Patienten erfolgt. Das Endgerät ist bevorzugt derart ausgebildet, dass eine solche Anleitung erfolgt, falls die Symptom-Abfrage ergeben hat, dass tatsächlich ein Kreislaufstillstand vorliegt.

Das Endgerät kann insbesondere derart ausgebildet sein, dass von ihm eine visuelle und/oder akustische Anleitung zur Thoraxkompression, Beatmung und ggf. Defibrillation erfolgt, wobei für eine Defibrillation ein eigenständiger Defibrillator beizubringen ist.

Der Lagesensor ist mit einem Trägermittel verbunden und zum Aufbringen (z. B. Aufkleben) auf die Haut des Patienten über dem Brustbein vorgesehen.

Das Trägermittel für den Lagesensor ist bevorzugt als Heftpflaster ausgebildet bzw. mit einem solchen Heftpflaster versehen, sodass der Lagesensor bei Bedarf auf unkomplizierte Art und Weise mittels Klebens an dem Patienten fixiert werden kann.

Das Heftpflaster bzw. Trägermittel kann ein selbstklebender, flexibler Streifen aus Gewebematerial und/oder Kunststoff sein, in welchem der Lagesensor integriert oder auf welchem der Lagesensor appliziert sein kann. Unter dem Begriff "Heftpflaster" wird hierin folglich ein (üblicherweise in der Medizin eingesetzter) einseitig zumindest teilweise mit einer Klebeschicht versehener Streifen aus z. B. Gewebematerial und/oder Kunststoff zur Befestigung auf der Haut eines Patienten verstanden.

Mit dem Trägermittel wird der Lagesensor an einer Position auf dem Brustbein an der Haut des Patienten fixiert (z. B. aufgeklebt) und mit dem Endgerät in Kommunikationsverbindung gebracht bzw. verbunden. Bei der Durchführung von Thoraxkompressionen können somit die damit einhergehenden Kompressionsbewegungen mittels des Lagesensors erfasst werden. Das mobile Endgerät kann insbesondere derart ausgebildet sein, dass von ihm mittels des Lagesensors die Eindrucktiefe (um die das Brustbein bei der Thoraxkompression eingedrückt wird) und/oder die Frequenz der Thoraxkompressionen erfasst werden und basierend auf diesen erfassten Werten die Anleitung des Helfers erfolgt.

Der Lagesensor kann z. B. ein Beschleunigungssensor sein, wobei aus den von dem Sensor erfassten Beschleunigungswerten sowohl die Eindrucktiefe wie auch die Kompressionsfrequenz errechnet werden können.

Die Erfindung ist anwendbar bei einem durch einen Kreislaufstillstand betroffenen Patienten, wobei der Ersthelfer, der insbesondere ein unerfahrener Laienhelfer sein kann, bei der Reanimation des Patienten und der Durchführung erforderlicher Maßnahmen, beispielsweise der Durchführung einer Herzdruckmassage, zeitnah unterstützt und fachgerecht angeleitet wird. Indem das erfindungsgemäße System unkompliziert aufgebaut und ohne Vorkenntnisse anwendbar ist, sind gesteigerte Erfolgsquoten bei derartigen Reanimationen ermöglicht.

Der mit dem Heftpflaster verbundene Lagesensor - auch als "Heftpflaster gegen den plötzlichen Herztod" bezeichnet - kann kostengünstig produziert werden, sodass eine Vielzahl davon einsetzbar und eine weite Verbreitung, beispielsweise an Plätzen mit großem Publikumsverkehr, in Sanitätskästen für Fahrzeuge oder bei Angehörigen von Menschen mit erhöhtem Risiko, einen plötzlichen Herzstillstand zu erleiden, möglich ist. Die Smartphone-App kann für die am weitesten verbreiteten Smartphone-Betriebssysteme (iOS, Android, Windows) zur Verfügung gestellt werden.

Die Smartphone-App und das "Heftpflaster gegen den plötzlichen Herztod" können helfen, bei Laienhelfern, hier besonders bei bisher noch nicht geschulten Laien, Ängste abzubauen und diesen wertvolle Hilfestellung bzw. Anleitung zur Wiederbelebung zu geben. Diese Maßnahmen können den Anteil und die Qualität der Laienreanimation erhöhen. Dies wiederum wird auch den Anteil derjenigen Patienten erhöhen, die ohne Behinderung überleben.

Gemäß einer Ausführungsform ist das Unterstützungssystem derart ausgebildet, dass von ihm die Anleitung des Helfers basierend auf den mittels des Lagesensors erfassten Messwerten durchgeführt wird. Dazu weist das mobile Endgerät eine (drahtlose oder drahtgebundene) Schnittstelle zum Übertragen der Messwerte des Lagesensors an das Endgerät auf.

Das Unterstützungssystem ist weiterhin bevorzugt derart ausgebildet, dass es dem Helfer ständig und in Echtzeit Rückmeldungen über die Qualität der Herzdruckmassage gibt und zu qualitativ hochwertiger Herzdruckmassage anleitet. Dazu kann das Endgerät z. B. derart ausgebildet sein, dass von ihm die mittels des Lagesensors erfassten Ist-Werte der Eindrucktiefe und/oder der Kompressionsfrequenz mit vorgegebenen Soll-Werten verglichen werden und bei Abweichung der Ist-Werte von den Soll-Werten eine entsprechende Korrekturanweisung ausgegeben wird.

Gemäß einer Ausführungsform ist das Unterstützungssystem derart ausgebildet, dass mittels Herstellens der Kommunikationsverbindung zwischen dem Endgerät und dem Lagesensor (z. B. mittels Anbindens des Lagesensors an das Endgerät) die vorstehend beschriebene Unterstützungsroutine (automatisch) gestartet wird. Demgemäß muss von dem Helfer zum Starten der Unterstützungsroutine keine zusätzliche Handlung vorgenommen werden.

Die Erfindung wird nachfolgend anhand eines Beispiels und einer Zeichnung näher erläutert.

An dem von einem Kreislaufstillstand betroffenen, liegenden Patienten 1 ist über seinem Brustbein 2 an der Haut 3 der Lagesensor 4 angeordnet, der mit dem als Heftpflaster 5 ausgebildeten Trägermittel verbunden und an der Haut 3 angeklebt ist.

Der Lagesensor 4 ist in diesem Beispiel als induktiv wirkender Bewegungssensor ausgebildet und steht gemäß der Ausführung mit einem als Smartphone ausgebildeten mobilen Endgerät 6 über eine Bluetooth-Schnittstelle 6.1 (nicht dargestellt) drahtlos in Verbindung.

Das Smartphone 6 ist mit einer Applikation (Smartphone-App) 7 (nicht näher dargestellt) als Anwendungsprogramm programmiert, das die von dem Lagesensor 4 ermittelten Daten und Symptome erfasst, aufbereitet und entsprechende Informationen einem auch als Laienhelfer 8 einsetzbaren Helfer (nicht dargestellt) zur Anleitung notwendiger Maßnahmen an dem Patienten 1 in geeigneter Weise, beispielsweise akustisch oder optisch in Textform oder als Video zur Verfügung stellt.

Somit erhält der Helfer bei allen nötigen Maßnahmen, wie Erkennen des Kreislaufstillstandes, Absetzen des Notrufs, Durchführen einfacher Hilfsmaßnahmen, Unterstützung durch die auf dem Smartphone 6 installierte Smartphone-App 7. In der App erfolgen sehr einfache und für Laien verständliche Abfragen nach den Symptomen des zu behandelnden Patienten 1, um festzustellen, ob ein Kreislaufstillstand vorliegt. Aus der App wird automatisiert ein Notruf bei der zuständigen Rettungsleitstelle abgesetzt; ferner wird über die in das Smartphone 6 integrierte Ortsbestimmung die Position desselben ermittelt und weitergeleitet.

Nun erfolgt die Anleitung des Laienhelfers 8 zur Durchführung der Thoraxkompressionen. Hierfür werden über den auf die Haut 3 des Patienten 1 über dem Brustbein 2 geklebten Lagesensor 4 die einzelnen Thoraxkompressionen gemessen. Das Feedbacksystem der Smartphone-App 7 gibt dem Helfer ständig und in Echtzeit Rückmeldung über die Qualität der Herzdruckmassage und leitet somit zu qualitativ hochwertiger Herzdruckmassage an. Besonderer Wert wird auf die Bedienergonomie der Software gelegt. Das Feedback der App 7 ist derart gestaltet, dass es für den Laienhelfer 8, der sich in einer extrem stressigen Situation befindet, einfach verständlich ist und die Anweisungen zur Verbesserung der Qualität leicht umsetzbar sind.

### Bezugszeichenliste

- 1: Patient
- 2: Brustbein
- 3: Haut
- 4: Lagesensor
- 5: Heftpflaster
- 6: mobiles Endgerät
- 6.1: Schnittstelle
- 7: Smartphone-App
- 8: Laienhelfer

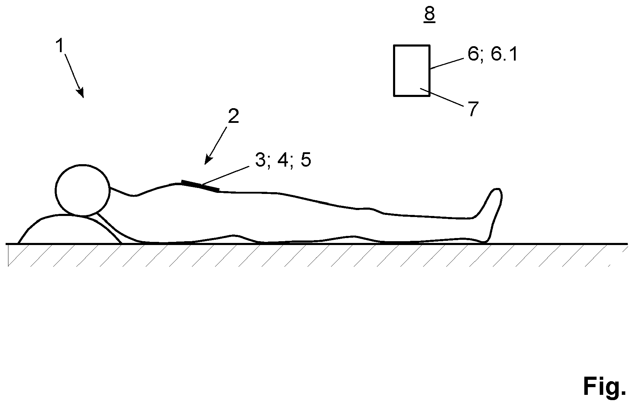

## Patentansprüche

1. System zur Unterstützung eines unausgebildeten Laienhelfers (8) bei der Durchführung von Thoraxkompressionen zur Reanimation eines von einem Kreislaufstillstand betroffenen Patienten (1), aufweisend einen Lagesensor (4) und ein mit dem Lagesensor (4) über eine Schnittstelle (6.1) in Verbindung stehendes, mobiles Endgerät (6) zur Erkennung eines Kreislaufzustandes des Patienten (1), wobei
- der Lagesensor (4) mit einem Heftpflaster (5) als Trägermittel verbunden ist, wobei das Heftpflaster (5) mit dem Lagesensor (4) auf der Haut (3) über dem Brustbein (2) des Patienten (1) fixierbar ist, wobei das Heftpflaster (5) ein in der Medizin eingesetzter, einseitig teilweise mit einer Klebeschicht versehener flexibler Streifen aus Gewebematerial und/oder Kunststoff ist, und wobei der Lagesensor (4) in das Heftpflaster (5) integriert ist,
- das mobile Endgerät (6) ein handelsübliches Smartphone ist, das derart ausgebildet ist, dass eigenständige Software-Applikationen ausführbar sind,
- das System eine Software-Applikation als Anwendungsprogramm zum Herstellen einer Kommunikationsverbindung zwischen dem Lagesensor (4), dem mobilen Endgerät (6) und dem Laienhelfer (8), zur Abfrage von durch den Laienhelfer (8) erfassbaren Symptomen, zur Auswertung der Symptome hinsichtlich der Feststellung des Kreislaufstillstands, als Anleitung zur Durchführung kurzfristig erforderlicher Maßnahmen zur Reanimation des Patienten (1) durch einen ungeschulten Laienhelfer (8) sowie zur Beschaffung und Analyse von mit dem Lagesensor (4) erfassten Daten umfasst,
- die Software-Applikation und das mobile Endgerät (6) zum automatisierten Absetzen eines Notrufs in Form einer Audio-, Video- oder Textnachricht an eine Rettungsleitstelle ausgebildet sind.

2. System nach Patentanspruch 1, **dadurch gekennzeichnet, dass** der Lagesensor (4) als induktiver Beschleunigungssensor ausgebildet ist.

3. System nach einem der Patentansprüche 1 bis 2, **dadurch gekennzeichnet, dass** das mobile Endgerät (6) über die Schnittstelle (6.1) drahtlos oder drahtgebunden mit dem Lagesensor (4) verbunden ist.

4. System nach einem der Patentansprüche 1 bis 3, **dadurch gekennzeichnet, dass** die Software-Applikation derart gestaltet ist, dass über Schnittstellen zum Betriebssystem und/oder zu einem Ortsbestimmungssystem des mobilen Endgeräts (6) eine Feststellung des aktuellen Standorts des Laienhelfers (8) durchführbar ist.

5. System nach einem der Patentansprüche 1 bis 4, **dadurch gekennzeichnet, dass** die Software-Applikation zur Ermittlung der Eindrucktiefe des Brustbeins (2) und der Beschleunigungswerte für die Kompressionsfrequenz der Thoraxkompression, zum Vergleich der ermittelten Ist- mit vorgegebenen Sollwerten und/oder deren Korrektur zur Anleitung für den Laienhelfer (8) ausgebildet ist.

6. System nach einem der Patentansprüche 1 bis 5, **dadurch gekennzeichnet, dass** der Notruf zeitgleich mit der Aktivierung der vom Lagesensor (4) erfolgten Symptomabfrage unabhängig vom Ergebnis derselben automatisiert auslösbar und absetzbar ist.

7. System nach einem der Patentansprüche 1 bis 5, **dadurch gekennzeichnet, dass** der Notruf in Abhängigkeit vom Ergebnis der Symptom-Abfrage absetzbar ist.
